# EUROPEAN PATENT SPECIFICATION

(11) **EP 0 270 007 B1**
(45) Date of publication and mention of the grant of the patent: **17.03.1993**
(21) Application number: 87117492.6
(22) Date of filing: 26.11.1987
(51) Int. Cl.: C07C 17/34, C07C 21/06

(54) **Process for producing vinylchloride monomer by pyrolysis of 1,2-dichloroethane**
Verfahren zur Herstellung von Vinylchloridmonomer durch Pyrolyse von 1,2-Dichloräthan
Procédé pour la fabrication du monomère de chlorure de vinyle par la pyrolyse d'1,2-dichloréthane

(30) Priority: 29.11.1986 JP 283083/86
(43) Date of publication of application: 08.06.1988
(73) Proprietor: TOSOH CORPORATION, Yamaguchi 746 (JP)
(72) Inventor: Teshima, Yutaka, Shinnanyo-shi Yamaguchi-ken (JP); Onishi, Satosi, Tokuyama-shi Yamaguchi-ken (JP)
(74) Representative: Grünecker, Kinkeldey, Stockmair & Schwanhäusser Anwaltssozietät

(56) References cited:
- EP-A- 0 014 920
- EP-A- 0 021 381
- EP-A- 0 180 925
- EP-A- 0 180 995
- DE-A- 2 913 030
- GB-A- 2 179 938
- PATENT ABSTRACT OF JAPAN, vol. 6, no. 239 (C-137)(1117), 26th November 1982 & JP-A-57142928

## Description

The present invention relates to a process for producing vinylchloride monomer (hereinafter designated as VCM) by pyrolysis of 1,2-dichloroethane (hereinafter designated as EDC) under pressure.

For the production of VCM it is known to introduce purified EDC in liquid form in a preheated zone of the pyrolysis furnace under pressure and, after evaporation in an evaporation zone, to thermally decompose same at a temperature of from 480 to 550°C in a pyrolysis reaction zone.

In this treatment the cracked gas which flows out of the pyrolysis furnace mostly contains VCM and hydrogen chloride produced by pyrolysis and EDC remaining undecomposed. Usually the abundant heat of the cracked gas at the high temperature is exhausted out of the system with cooling water in a quencher which follows in the operation line. In the next step of the operation, hydrogen chloride and the EDC remaining undecomposed are separated from VCM by use of a distillation column.

While the high temperature pyrolysis furnace for EDC is operated for a long period of time, rigid coke is generally formed in the inside of the tube line. Therefore it is necessary to stop the operation before the pressure loss of the pyrolysis furnace itself exceeds the tolerance limit and decoke.

The rate of pyrolysis of EDC, or the yield of VCM per unit amount of EDC introduced in the pyrolysis furnace, can be enhanced by elevating the temperature at which the pyrolysis reaction takes place. As a result of the treatment, however, by-products such as methylchloride and butadiene which may cause trouble in polymerization are also increased. These by-products require a large amount of energy to be removed in the following stage.

Further, in the pyrolysis furnace where EDC is preheated, evaporated and decomposed by heat, the elevated temperature inside the furnace promotes the formation of rigid coke on the inside wall of the tubes, which consequently shortens the life of the pyrolysis furnace.

In addition, the elevated temperature at which the exhaust gas after combustion is exhausted from the pyrolysis furnace brings about a large increase in the loss of thermal energy.

For these reasons, the temperature of the pyrolysis reaction of EDC in the actual operations is limited by the amounts of by-products and coke which may be formed in parallel. In other words, the rate of pyrolysis can hardly be increased beyond a certain level.

For reducing the amount of coking formed in the tubes of the pyrolysis furnace, Japanese Laid-Open Patent Application No. Sho 49-125306 proposes a process in which EDC is evaporated at the outside of the pyrolysis furnace or, in other words, gaseous EDC is introduced into the pyrolysis furnace.

This process, however, requires a high temperature heat source for the evaporation of EDC and therefore a heating furnace for EDC is needed in addition to the pyrolysis furnace.

Further, additional problems of the heating furnace itself are the deterioration of EDC due to the high temperature and the necessity for removing the scales. Thus, the expense for the operation and equipment are necessarily a loss in the cost.

On the other hand, the cracked gas flowing out of the pyrolysis furnace has a tremendous amount of heat energy, which is lost to the cooling water in a quencher located immediately after the pyrolysis furnace.

Therefore, a variety of methods have been proposed for recovering and utilizing the energy of the high temperature cracked gas flowing out of the pyrolysis furnace.

For instance, Japanese Laid-Open Patent Application No. Sho 56-45424 describes a method in which a cracked gas flowing out of a pyrolysis furnace is cooled at least in two steps and the heat transferred to a heat transfer medium is used to heat other devices.

The heat transfer medium should be selected by considering the resistance against the high temperature deterioration and the specific property of the gas flowing out of the pyrolysis furnace.

In the worst case explosion due to leakage of hydrogen chloride or others contained in the gas occurs.

In particular, when water or steam is used as heat transfer medium, the leakage of hydrogen chloride may probably cause severe corrosion and even breakage of devices.

In this case, the apparatus should be constructed in a larger scale, to utilize thus recovered heat.

DE-A-2913030 discloses a process in which heat of a cracked gas is indirectly exchanged using a heat transfer medium and the heat transfer medium is employed as a heat source of a distillation column for EDC, as an example of using the heat transfer medium for a heat source in another operation.

Here, a troublesome operation is usually required, for example, because the distillation column is under the influence of the variation in the running conditions of the pyrolysis furnace. Furthermore, since usually a pyrolysis furnace is situated some distance apart from a distillation column, the above operation necessarily requires a large expense for the equipment.

Furthermore, another process is known in which the exchange of heat between a cracked gas flowing from a pyrolysis furnace and EDC supplied to the pyrolysis furnace is utilized to preheat or evaporate the EDC.

In this case, however, the cracked gas flows out at a relatively high rate and therefore a fairly large length is necessary for the heat transmission tube of the heat exchangers. Consequently the pressure drop in the tubes occurs in the heat exchanger which is located immediately after the pyrolysis furnace and this entails to shorten the time for a continuous operation of the pyrolysis furnace. Thus, to overcome the difficulties, a large scale remodeling of the pyrolysis furnace is necessary, for example, to expand the diameter or shorten the length of the heat transmission tubes.

In order to raise the rate of pyrolysis without elevating the temperature of the pyrolysis reaction, or in other words without causing an increase of the amount of by-products formed in the pyrolysis furnace and a loss in thermal energy, it is necessary to increase the area of the reaction zone in the pyrolysis furnace without any drastic remodeling of the furnace. Further, for the reduction of coking in tubes of the pyrolysis furnace, gaseous EDC should be introduced in the pyrolysis furnace at a site which lies in a range of zones called for liquid EDC from the preheating zone to the evaporation zone, preferably at the entrance of the preheating zone.

The cracked gas flowing out of the pyrolysis furnace contains a large amount of thermal energy which is recovered by exchanging heat between the cracked gas and the supplied EDC and utilized for preheating and evaporating the EDC.

However, the mentioned task requires an effective heat exchanger which is installed immediately after the pyrolysis furnace and performs the exchange of heat between the cracked gas and the supplied EDC with an operational condition set so as to minimize the pressure drop.

Further, it is also necessary for a long operation to prevent the continuous operation of the pyrolysis furnace from being interrupted by a lowered capacity of the heat exchanger, an increased pressure drop, and a blocking of the heat exchanger due to scaling.

It is the object of the present invention to provide a process for producing vinylchloride monomer wherein the pressure drop of the heat exchanger in which EDC introduced in the pyrolysis furnace is preheated and evaporated by the heat exchanged from the cracked gas at a high temperature is minimized and the amount of coke to be formed and attached onto the heat exchanger while the device is operated is reduced. Said object is achieved by a process for producing vinylchloride monomer by pyrolysis of 1,2-dichloroethane comprising carrying out a heat exchange between a high temperature cracked gas flowing out of a pyrolysis furnace and the 1,2-dichloroethane to be introduced into the pyrolysis furnace with a flow rate of said cracked gas of from 5 m/s to less than 20 m/s to cool said cracked gas to 180 - 350°C and introducing gaseous 1,2-dichloroethane heated by the heat exchange into a preheating zone or a zone anterior thereto in the pyrolysis furnace.

The present invention will be explained in further details.

The exchange of heat between a high temperature cracked gas flowing out of the pyrolysis furnace and the EDC to be introduced in the pyrolysis furnace may be performed by any process, but preferably the heat exchanger is so designed that the gas is forced to flow in a heat transmitting tube and the tube is immersed in the incoming liquid EDC.

The procedure mentioned above is conducted at a flow rate of the cracked gas ranging from 5 m/s to less than 20 m/s.

When the flow rate of the cracked gas is less than 5 m/s, scaling mainly of carbon is accelerated on the inside wall of the tube, which adversely affects the heat exchanger and in the worst case the device may become inoperable on account of blockage of the tube.

When the flow rate of the cracked gas is 20 m/s or higher, the pressure drop in the tube which is caused by the high rate of flow should be taken into consideration.

As far as the above conditions are satisfied, any type of heat exchanger may be employed, but preferably the heat exchanger is of a so-called mono-tube type.

Further the cracked gas is cooled to 180 - 350°C. If the gas is cooled to a temperature below 180°C, an undesired condensation and a lowered flow rate of the gas may occur, which may eventually cause the complete blocking of the tube. On the other hand, if the gas is cooled to more than 350°C, insufficient heat is recovered from the high temperature cracked gas and therefore additional heat should be supplied for evaporation of the EDC by means of steam or another source of heating. This brings about an economical disadvantage.

Liquid EDC to be supplied to the heat exchanger and to be eventually decomposed should be at a temperature between 160°C and 250°C.

A temperature of the EDC below 160°C is undesirable, because it may cause cooling of the cracked gas to less than 180°C which is to be avoided as mentioned above.

The exchange of heat which performs cooling of the cracked gas and preheating and evaporation of EDC to be supplied to the pyrolysis furnace can be carried out with a single heat exchanger. However, as far as the above conditions are met, the same object can be attained with plural separate devices, one of which is a heat exchanger which mostly preheats the EDC and the others are two or more heat exchangers which mostly contribute to the evaporation of the EDC.

The gaseous EDC flowing out of the heat exchanger does not contain much mist or tiny drops of liquid EDC. It is introduced in the pyrolysis furnace at the site which ranges from the preheating to the evaporating zone, preferably at the preheating zone, for liquid EDC.

In this manner, the gaseous EDC can be introduced in the pyrolysis furnace without making any change or with a slight change on the previous pyrolysis furnace, to permit the reaction zone to be enlarged in the pyrolysis furnace.

The enlarged area of the zone described above for the pyrolysis reaction increases the rate of pyrolysis of EDC or, in other words, the amount of VCM produced per unit amount of EDC supplied is increased by approximately 5 - 10% without any increase of the amount of undesired by-products, any elevation of temperature after combustion of an exhaust gas from the pyrolysis furnace, and any consequent increase of loss in thermal energy.

Further, the amount of coke produced, or the concurrent pressure drop, in the pyrolysis furnace is also decreased by 70 - 90% less than compared with the case when EDC in liquid form is introduced in the pyrolysis furnace.

After a long time operation of the heat exchanger to evaporate liquid EDC, the decrease in the purity of the EDC remaining in the heat exchanger is negligibly small and the gaseous EDC introduced in the pyrolysis furnace is as pure as the liquid EDC supplied to the heat exchanger.

This fact can be markedly observed when a part, or more particularly 5 - 10%, of liquid EDC supplied to the heat exchanger is extracted as it is from the bottom of the heat exchanger and 95 - 90% is evaporated and introduced in the pyrolysis furnace.

Even after a long time operation, no decrease in the purity is observed with the EDC remaining in the heat exchanger and the gaseous EDC to be introduced in the pyrolysis furnace.

In the heat exchanger in which the exchange of heat is carried out between EDC to be supplied to the pyrolysis furnace and a high temperature cracked gas flowing out of the pyrolysis furnace, it was feared at the part of the device where EDC was being evaporated that coking may cause a decrease of the heat transmission and further disturb the operation. Surprisingly no bad influence was observed even if scales were formed. They can be readily peeled off so as to not disturb the operation.

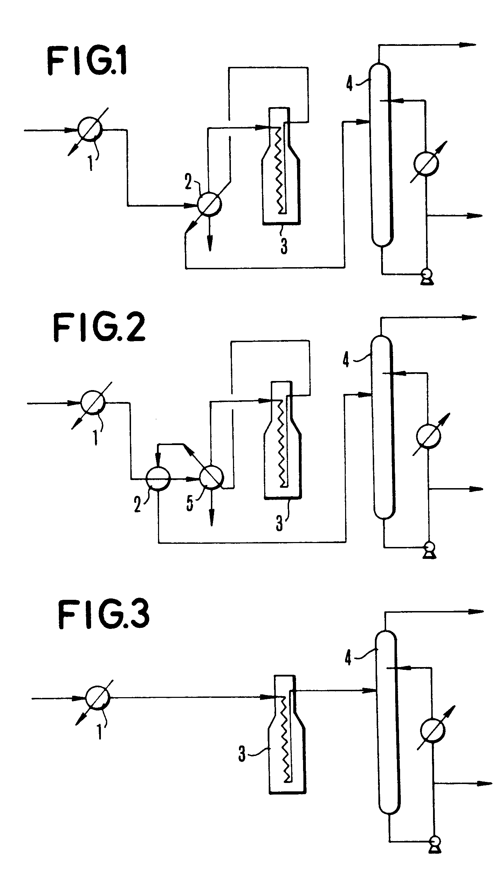

Figs. 1 and 2 are flow sheets of an apparatus used in the process of the present invention and Fig. 3 is a flow sheet of an apparatus used in the process of the comparison Example.

The present invention is further illustrated by some examples.

Pressures are expressed by the pressure on guage and percentages (%) for the proportions are by weight, if not otherwise specified.

### Example 1

Employing an apparatus shown in Fig. 1, a single heat exchanger 2 served to cool a high temperature cracked gas from a pyrolysis furnace 3 and to preheat and evaporate EDC to be introduced into the pyrolysis furnace 3.

In this treatment, 8,600 kg/h of liquid EDC being under a pressure of 3,600 kPa (36 atm) was preheated to 190°C in a heat exchanger 1 and supplied to the bottom of the heat exchanger 2.

On the other hand, 8,600 kg/h of a cracked gas flowing out of the pyrolysis furnace 3, at 500°C and 2,300 kPa (23 atm) was delivered at a flow rate of 9.2 - 13.8 m/s into the tube-side of the heat exchanger 2, to exchange heat with the fresh EDC.

Through the procedure above, the cracked gas leaving the heat exchanger 2 was at 245°C and further cooled to 80°C in the quencher 4 before being passed to the next step of operation.

The total amount of the EDC supplied was evaporated and turned into a vapor at 265°C, which was then delivered to the pyrolysis furnace 3 at the part previously called preheating zone, so as to be decomposed thermally.

The cracked gas contained 3,200 kg/h of VCM and also butadiene and methylchloride in amounts of 4.3 and 35 ppm by weight, respectively, per unit VCM. After a continuous operation for 95 days conducted under the above-mentioned condition, the increase in the pressure drop of the pyrolysis furnace 4 was 1.3 times as much owing to the coking in the inside of tubes of the pyrolysis furnace 4.

Further, scales of which carbon molecules were the main ingredient were formed on the outer surface of the tube in the heat exchanger 2 with which the liquid EDC was contacted, but most of the scales were peeled off. A small amount of coking could be found attached to the inside of the tube at the inlet part. The cracked gas contacted the whole inside wall of the tube, but the metal surface remained uncoated except only at the inlet part. The capacity of operation was lowered by not more than 10% of the original one and therefore no obstacle was observed for the operation.

The expenditure of energy from the heat exchanger 1 through the pyrolysis furnace 3 amounted to 71% of that estimated in Comparison Example 1 of this specification.

### Example 2

The following procedure was carried out using the apparatus shown in Fig. 2.

Liquid EDC in an amount of 8,600 kg/h under an applied pressure 3,600 kPa (36 atm) was preheated to 160°C with steam in a heat exchanger 1 and then passed to a heat exchanger 2 through which 8,600 kg/h of a cracked gas of 235°C coming from the pyrolysis furnace 3 through a heat exchanger 5 for the first step cooling.

The whole amount of liquid EDC heated to 190°C in the heat exchanger 2 was further introduced in the bottom of the heat exchanger 5.

The cracked gas which suffered from the second cooling in the heat exchanger 2 was at a temperature of 210°C and this was passed to a conventional quencher 4 to be cooled to 80°C.

The flow rate of the cracked gas was controlled in a range from 8.3 to 13.8 m/s in the heat exchanger.

The EDC introduced in the heat exchanger 5 experienced an exchange of heat with the cracked gas of 500°C and 2,300 kPa (23 atm) and was evaporated into a vapor of 265°C. This was then transferred to the pyrolysis furnace 3 at the site of the preheating zone as in convention processes, to perform the pyrolysis.

In this process 3,170 kg/h of VCM could be obtained in the pyrolysis furnace 3 which contained 3.6 and 32 ppm by weight of butadiene and methylchloride, respectively, per unit VCM.

Expensive fuel supplied to the pyrolysis furnace 3 was 67% as much as that appearing in Comparison Example 1 of this specification. After a continuous operation for about 70 days conducted under the same condition, the increase of the pressure drop in the pyrolysis furnace 3 due to the coke which deposited on the inside wall of tubes in the pyrolysis furnace 3 proved to be approximately 1.3 times as much.

Further, scales of which carbon molecules were the main ingredient were formed on the outer surface of the tube in the heat exchangers 2 and 5 with which the liquid EDC was contacted, but most of the scales were peeled off. A small amount of coking could be found attached to the inside of the tube at the inlet part. The cracked gas contacted the whole inside wall of the tube, but the metal surface remained uncoated except only at the inlet part. The capacity of operation was lowered by not more than 10% of the original one and therefore no obstacle was observed for the operation.

After a 70 day operation, the liquid EDC remaining in the heat exchanger 5 which was concerned mainly with the evaporation of EDC changed its own concentration by 0.5% less than that of the originally supplied EDC, but only a negligible influence was observed on the pyrolysis reaction.

### Comparison Example 1

Decomposition of EDC was carried out using an apparatus shown in Fig. 3.

Liquid EDC in an amount of 8,600 kg/h was preheated to 160°C with steam in a heat exchanger 1 under an applied pressure of 3,300 kPa (33 atm) and delivered as it was in the liquid form to a pyrolysis furnace 3 at the preheating zone for EDC.

In this manner, the liquid EDC supplied was heated to about 260°C in the tube of the pyrolysis furnace 3 and then completely evaporated in the evaporation zone and further incompletely decomposed by heat in the pyrolysis reaction zone.

The gas produced by the pyrolysis reaction had a temperature of 510°C just after the pyrolysis. This was directly passed to a quencher 4 and cooled there to a temperature of 80°C before being transferred to the next step of operation.

The gas produced by the pyrolysis reaction contained 2,960 kg/h of VCM and further butadiene and methylchloride in amounts of 6 and 40 ppm by weight, respectively, per unit VCM.

After a continuous operation for 82 days under the same same condition, rigid coke was formed and attached to the inside of the tube of the pyrolysis furnace 3, which lead to about 1.5 times as much pressure drop as that at the original stage with the pyrolysis furnace 3.

## Claims

1. A process for producing vinylchloride monomer by pyrolysis of 1,2-dichloroethane comprising carrying out a heat exchange between a high temperature cracked gas flowing out of a pyrolysis furnace and the 1,2-dichloroethane to be introduced into the pyrolysis furnace with a flow rate of said cracked gas of from 5 m/s to less than 20 m/s to cool said cracked gas to 180 - 350°C and introducing gaseous 1,2-dichloroethane heated by the heat exchange into a preheating zone or a zone anterior thereto in the pyrolysis furnace.

## Patentansprüche

1. Verfahren zum Herstellen von Vinylchloridmonomer durch Pyrolyse von 1,2-Dichlorethan, welches das Durchführen eines Wärmeaustausches zwischen einem bei hoher Temperatur gecrackten Gas, welches aus einem Pyrolyseofen fließt, und dem 1,2-Dichlorethan, welches in den Pyrolyseofen eingeführt wird, mit einer Fließgeschwindigkeit des gecrackten Gases von 5 m/s bis weniger als 20 m/s, um das gecrackte Gas auf 180 - 350 °C zu kühlen und das Einführen des gasförmigen 1,2-Dichlorethans, welches durch den Wärmeaustausch erwärmt ist, in eine Vorwärmzone oder eine vorhergehende Zone in den Pyrolyseofen umfaßt.

## Revendications

1. Procédé pour la fabrication du monomère de chlorure de vinyle par pyrolyse de 1,2-dichloréthane, comprenant l'exécution d'un échange de chaleur entre un gaz craqué à haute température sortant d'un four de pyrolyse et le 1,2-dichloréthane devant être introduit dans le four de pyrolyse à un débit dudit gaz craqué de 5 m/s à moins de 20 m/s afin de refroidir ledit gaz craqué à 180 - 350°C et l'introduction du 1,2-dichloréthane gazeux chauffé par l'échange de chaleur dans une zone de préchauffage ou une zone antérieure à celle-ci dans le four de pyrolyse.
